# EUROPÄISCHE PATENTANMELDUNG

(11) **EP 3 932 312 A1**
(43) Veröffentlichungstag der Anmeldung: **05.01.2022**
(21) Anmeldenummer: 21182624.3
(22) Anmeldetag: 30.06.2021
(51) Int. Cl.: A61B 5/279, A61B 5/00, A61B 5/268, A61B 5/27, A61N 1/04, A41D 13/12

(54) **ANZIEHBARE VORRICHTUNG UND TROCKENELEKTRODE ZUR ERFASSUNG ELEKTROPHYSIOLOGISCHER SIGNALE SOWIE VERFAHREN ZUR HERSTELLUNG DERSELBEN**

(30) Priorität: 30.06.2020 DE 102020208157
(71) Anmelder: Fraunhofer-Gesellschaft zur Förderung der angewandten Forschung e.V., 80686 München (DE)
(72) Erfinder: Rybalko, Ruslan, 91058 Erlangen (DE); Hofmann, Christian, 91058 Erlangen (DE)
(74) Vertreter: Schlenker, Julian

(57) **Zusammenfassung**

Ausführungsbeispiele schaffen eine anziehbare Vorrichtung zur Erfassung elektrophysiologischer Signale eines Lebewesens. Die Vorrichtung umfasst einen textilen Träger, zumindest zwei Trockenelektroden, die auf einer Innenseite des textilen Trägers angebracht sind, und zumindest zwei an dem textilen Träger angebrachten, einstellbaren Gurten, die es ermöglichen, die anziehbare Vorrichtung auf einen Körper des Lebewesens und einen Anpressdruck der zumindest zwei Trockenelektroden auf eine Haut des Lebewesens einzustellen.

## Beschreibung

Ausführungsbeispiele der vorliegenden Erfindung beziehen sich auf eine anziehbare Vorrichtung zur Erfassung elektrophysiologischer Signale. Weitere Ausführungsbeispiele beziehen sich auf eine Trockenelektrode zur Erfassung elektrophysiologischer Signale. Weitere Ausführungsbeispiele beziehen sich auf ein Verfahren zur Herstellung einer anziehbaren Vorrichtung zur Erfassung elektrophysiologischer Signale.

Für die Erfassung elektrophysiologischer Signale an Lebewesen, wie z.B. am menschlichen Körper, ist eine gute Kontaktierung der primäre Messwertaufnehmer (z.B. Elektroden) notwendig, damit eine gute Signalqualität erzielt werden kann. Darüber hinaus muss diese Adaption auch eine ausreichende Robustheit aufweisen, da bei Signalerfassung im Alltag und unter Bewegung sonst Störungen durch nicht gleichmäßig anliegende Elektroden auftreten (Bewegungsartefakte).

Wird die Signalverarbeitungskette genauer betrachtet, so sind für die Signalerfassung vom Körper bis zu einer Auswerteelektronik verschiedene Funktionsblöcke miteinander verknüpft:
▪ der Elektrodenbereich muss leitfähig sei und einen möglichst geringen Widerstand aufweisen, um die physiologischen Signale in guter Qualität aufzunehmen,
▪ der Leitungsabschnitt soll die aufgenommenen Messwerte möglichst störungsfrei bis zur Kontaktierungsstelle mit der Auswerteelektronik übertragen, und
▪ alle verwendeten Komponenten und Materialien sollen einen hohen Tragekomfort und Biokompatibilität aufweisen.,

Im Sinne einer guten Adaption sollen die Messwertaufnehmer auch bei unterschiedlichen Körpergrößen, unterschiedlichen Körperproportionen und für verschiedene Geschlechter eine gute Signalerfassung gewährleisten. Daher müssen textile Trägersysteme mit integrierten Messwertaufnehmern (z.B. Shirt oder Weste oder Gurt) diesen anatomischen Anforderungen Rechnung tragen.

Bei der Erfassung elektrophysiologischer Signale am Körper werden im klinischen Alltag typischerweise Silber-Silberchlorid-Klebeelektroden (Ag/AgCI) verwendet. Diese Art von

Elektroden verfügen über einen Kleberand (ähnlich wie bei einem Pflaster) und einen leitfähigen Kern, der zusätzlich mit einem Kontaktgel ausgeführt ist, damit ein geringer Haut-Elektroden-Widerstand erreicht werden kann.

Diese Elektroden haben jedoch die folgenden Nachteile:
▪ Beim Anlegen der Elektroden fühlt sich das Gel für die zu vermessenden Personen unangenehm an.
▪ Mit längerer Messdauer verliert der Kleberand aufgrund der natürlichen Transpiration seine Klebewirkung, wodurch die Elektroden abfallen können und aufgrund von Signalverlust keine auswertbaren Daten mehr aufgenommen werden können.
▪ Bei längerer Anwendung (z.B. 24h-EKG (EKG = Elektrokardiogramm) oder Messung bis zu einer Woche) können Hautirritationen auftreten.

Seit einigen Jahren werden verschiedene Materialien für Trockenelektroden erforscht, die aufgrund einer hohen Leitfähigkeit den Einsatz von Kontaktgel vermeiden. Diese Trockenelektroden sind in den allermeisten Fällen in textile Trägersysteme eingearbeitet (z.B. Pulsbrustgurt, Sensorshirt, Weste, Gurtsystem). Sensorsysteme für ein Ein-Kanal-EKG können recht einfach umgesetzt werden. Hier werden zwei Elektroden z.B. in einen Brustgurt integriert, der nahezu immer gleichmäßig am Körper anliegt und stabile Signale liefert. An Sensorsysteme zur Aufzeichnung von Mehrkanal-EKGs sind deutliche höhere Anforderungen gestellt, da hier mehrere Elektroden (z.B. vier Elektroden für ein Dreikanal-EKG bzw. sieben Elektroden für ein Neuen-Kanal-EKG bzw. zehn Elektroden für ein zwölf-Kanal-EKG) in das Textil an unterschiedlichen Applikationsstellen zu integrieren sind und dabei immer eine ausreichend gute Adaption gewährleistet werden muss.

Dabei müssen die textilen Träger immer auch den schon oben erwähnten anatomischen Anforderungen der Trägerinnen oder Träger gerecht werden (z.B. Geschlecht, Größe, Proportionen, Einschränkungen).

Nachteile bisheriger textilintegrierter Sensorsysteme:
▪ Keine ausreichend gute Signale wegen zu schlechter Elektrodenmaterialien.
▪ Keine ausreichend gute Signale wegen zu geringer Adaption (z.B. kein ausreichend hoher Anpressdruck).
▪ Keine ausreichend guten Signale wegen Bewegungsartefakten.
▪ Verwendete Konstruktion und Materialien nicht waschbar, keine Aufbereitung möglich.
▪ Keine Langzeitstabilität der Signalqualität (Leitwert des Elektrodenmaterials nimmt ab, z.B. bei Waschvorgängen) Qualität der.
▪ Spezifische Ausführung für Frauen und Männer notwendig.
▪ Viele verschiedene Konfektionsgrößen notwendig.
▪ Sensorsystem zu auffällig und daher nicht akzeptiert.
▪ Sensorsystem unkomfortabel im Alltag zu tragen.
▪ Sensorsystem unkomfortabel bei Schlafanalyse zu tragen.
▪ Sensorsystem nicht einstellbar.
▪ Sensorsystem nicht allein anziehbar.
▪ Sensorsystem führt zu starken Einschränkungen bei der Kleiderwahl.

Ein wichtiger Punkt beim Monitoring im Alltag ist die ausreichende Akzeptanz und gute Anwendbarkeit durch die Nutzer. Die Anwender müssen die textilen Trägersysteme einfach anlegen und im Alltag jederzeit schnell und unkompliziert anpassen können.

Der vorliegenden Erfindung liegt daher die Aufgabe zugrunde die bestehende Situation zu verbessern.

Diese Aufgabe wird durch die unabhängigen Patentansprüche gelöst.

Vorteilhafte Weiterbildungen finden sich in den abhängigen Patentansprüchen.

Ausführungsbeispiele schaffen eine anziehbare Vorrichtung zur Erfassung elektrophysiologischer Signale eines Lebewesens [z.B. Tieres oder Menschen [z.B. menschlichen Patienten]]. Die Vorrichtung umfasst einen textilen Träger, zumindest zwei Trockenelektroden [z.B. zwei, drei oder vier Trockenelektroden], die auf einer Innenseite des textilen Trägers angebracht sind, und zumindest zwei an dem textilen Träger angebrachten, einstellbaren Gurten, die es ermöglichen, die anziehbare Vorrichtung auf einen Körper des Lebewesens und einen Anpressdruck der zumindest zwei Trockenelektroden auf eine Haut des Lebewesens einzustellen.

Bei Ausführungsbeispielen können die zumindest zwei Trockenelektroden zumindest vier Trockenelektroden zur Erfassung eines mehrkanaligen Elektrokardiogramms des Lebewesens sein.

Bei Ausführungsbeispielen kann der textile Träger aus einem [z.B. einzigen] Materialstück gefertigt sein.

Bei Ausführungsbeispielen kann der textile Träger zusammen mit den zumindest zwei Gurten die Form eines Gurtsystems bilden.

Bei Ausführungsbeispielen kann der textile Träger einen mittleren Bereich, zwei obere Bereiche, die sich jeweils von dem mittleren Bereich weg erstrecken, und zwei seitliche Bereiche, die sich jeweils von dem mittleren Bereich weg erstrecken, aufweisen.

Bei Ausführungsbeispielen kann sich, wenn die Vorrichtung von dem Lebewesen getragen wird, der mittlere Bereich des textilen Trägers über einen Rückenbereich des Lebewesen erstreckt, die zwei oberen Bereiche des textilen Trägers ausgehend von dem Rückenbereich über jeweilige Schulterbereiche bis hin zu oberen Brustbereichen des Lebewesen erstrecken, und die zwei seitlichen Bereiche des textilen Trägers ausgehend von dem Rückenbereich über jeweilige Axillarlinien bis hin zu jeweiligen oberen Bauchbereichen oder unteren Brustbereichen erstrecken.

Bei Ausführungsbeispielen können die zwei oberen Bereiche des textilen Trägers mit den zwei seitlichen Bereichen des textilen Trägers über zwei der zumindest zwei Gurte verbindbar sein.

Bei Ausführungsbeispielen können die zwei seitlichen Bereiche des textilen Trägers über ein Verbindungselement [z.B. Klettverschluss, Steckschnalle, Klemmriemen oder Druckknopf] miteinander verbindbar sein.

Bei Ausführungsbeispielen können die zwei seitlichen Bereiche des textilen Trägers über einen dritten Gurt der zumindest zwei Gurte miteinander verbindbar sein.

Bei Ausführungsbeispielen können die zumindest zwei Trockenelektroden an zumindest zwei Bereichen aus den zwei oberen Bereichen und den zwei seitlichen Bereichen des textilen Trägers an dem textilen Träger angebracht sein.

Bei Ausführungsbeispielen können die zumindest zwei Trockenelektroden zumindest vier Trockenelektroden sein, wobei zwei Trockenelektroden der zumindest vier Trockenelektroden auf einer Innenseite der zwei oberen Bereiche des textilen Trägers angebracht sind, wobei zwei andere Trockenelektroden der zumindest vier Trockenelektroden auf einer Innenseite der zwei seitlichen Bereiche des textilen Trägers angebracht sind.

Bei Ausführungsbeispielen können die zwei Trockenelektroden, wenn die Vorrichtung von dem Lebewesen getragen wird, Schlüsselbeinbereiche [z.B. einen linken und rechten Schlüsselbeinbereich] oder obere Bereiche oberhalb eines Brustbereich des Lebewesens kontaktieren.

Bei Ausführungsbeispielen können die zwei anderen Trockenelektroden, wenn die Vorrichtung von dem Lebewesen getragen wird, abdominale Bereiche [z.B. innerhalb eines abdominalen Quadranten] oder untere Bereiche unterhalb eines Brustbereichs des Lebewesens kontaktieren.

Bei Ausführungsbeispielen können der textile Träger und/oder die zumindest zwei Gurte elastisch sein.

Bei Ausführungsbeispielen können die zumindest zwei Trockenelektroden jeweils eine Schicht aus leitfähigem Gewebe [z.B. versilbertem Gewebe] und eine Schicht aus elektrisch leitfähigem Polymer [z.B. Silikon], die die Schicht aus leitfähigem Gewebe bedeckt, aufweisen.

Bei Ausführungsbeispielen kann die Schicht aus elektrisch leitfähigem Polymer dünner sein als 1 mm.

Bei Ausführungsbeispielen können [z.B. verschiedene] Schichten der zumindest zwei Trockenelektroden mittels einer Kombination aus einem Siebdruckverfahren [z.B. zum Vorbereiten der einzelnen Funktionsschichten] und einem Transferdruckverfahren [z.B. zum Packaging = Zusammenschweißen und Integration in den textilen Träger] gebildet sein.

Bei Ausführungsbeispielen können die zumindest zwei Trockenelektroden über isolierte Leitungen mit einem an einer Außenseite des textilen Trägers angebrachten Anschluss [z.B. zum Bereitstellen der mit den zumindest zwei Trockenelektroden erfassten elektrophysiologischen Signale] verbunden sein.

Bei Ausführungsbeispielen können die isolierten Leitungen jeweils eine Schicht aus leitfähigem Gewebe und zumindest eine Schicht aus Isolationsmaterial [z.B. nur Deckschicht oder auch Grund- und Deckschicht], die die Schicht aus leitfähigem Gewebe bedeckt, aufweisen.

Bei Ausführungsbeispielen können Schichten der isolierten Leitungen mittels einer Kombination aus einem Siebdruckverfahren und einem Transferdruckverfahren gebildet sein.

Bei Ausführungsbeispielen können die isolierten Leitungen jeweils mit einem nach außen geführten Anschlusselement [z.B. Druckknopf, Bananenbuchse] des Anschlusses verbunden sein.

Bei Ausführungsbeispielen kann der Anschluss zumindest eine Schicht aus Isolationsmaterial [z.B. nur Deckschicht oder auch Grund- und Deckschicht] aufweisen, wobei eine Schicht aus der zumindest einen Schicht aus Isolationsmaterial in Bereichen der Anschlusselemente geöffnet ist, so dass die Anschlusselemente frei liegen.

Weitere Ausführungsbeispiele schaffen eine Trockenelektrode zur Erfassung elektrophysiologischer Signale eines Lebewesens. Die Trockenelektrode umfasst eine Schicht aus leitfähigem Gewebe [z.B. versilbertem Gewebe], und einer Schicht aus elektrisch leitfähigem Polymer [z.B. Silikon], die die Schicht aus leitfähigem Gewebe bedeckt.

Bei Ausführungsbeispielen kann das leitfähige Gewebe ein versilbertes Gewebe sein.

Bei Ausführungsbeispielen kann die Schicht aus elektrisch leitfähigem Polymer [z.B. Silikon] eine Dicke von Weniger als 1 mm aufweisen.

Bei Ausführungsbeispielen kann die Trockenelektrode ferner eine thermoplastische Polyurethanfolie aufweisen, wobei die Schicht aus leitfähigem Gewebe auf der thermoplastischen Polyurethanfolie angeordnet ist.

Bei Ausführungsbeispielen kann die Trockenelektrode in einem Transferdruckfolienschichtsystem aus zumindest zwei Transferdruckfolien eingebettet sein, wobei das Transferdruckfolienschichtsystem in einem zu der Schicht aus elektrisch leitfähigem Polymer [z.B. Silikon] benachbarten Bereich teilweise geöffnet ist, so dass die Schicht aus elektrisch leitfähigem Polymer teilweise freiliegt.

Bei Ausführungsbeispielen kann die Trockenelektrode mittels eines Transferdruckverfahrens auf einem textilen Träger angebracht sein.

Weitere Ausführungsbeispiele schaffen ein Verfahren zur Herstellung einer anziehbaren Vorrichtung zur Erfassung elektrophysiologischer Signale eines Lebewesens. Das Verfahren umfasst einen Schritt des Bereitstellens eines textilen Trägers. Ferner umfasst das Verfahren einen Schritt des Bildens von zumindest zwei Trockenelektroden auf einer Innenseite des textilen Trägers mittels eines Transferdruckverfahrens. Ferner umfasst das Verfahren einen Schritt des Bereitstellen von zumindest zwei einstellbaren Gurten und Anbringen der zumindest zwei einstellbaren Gurte an den textilen Träger.

Bei Ausführungsbeispielen kann das Bilden der zumindest zwei Trockenelektroden einen Schritt des Bereitstellens einer Schicht aus elektrisch leitfähigem Gewebe [z.B. versilbertem Gewebe], und einen Schritt des Bereitstellens einer Schicht aus elektrisch leitfähigem Polymer [z.B. Silikon] auf der Schicht aus elektrisch leitfähigem Gewebe, so dass die Schicht aus elektrisch leitfähigem Polymer die Schicht aus elektrisch leitfähigem Gewebe zumindest teilweise bedeckt, aufweisen.

Bei Ausführungsbeispielen kann das Bilden der zumindest zwei Trockenelektrode ferner einen Schritt des Bereitstellens einer thermoplastischen Polyurethanfolie aufweisen, wobei die Schicht aus leitfähigem Gewebe auf der thermoplastischen Polyurethanfolie angeordnet ist.

Bei Ausführungsbeispielen kann das Bilden der zumindest zwei Trockenelektroden ferner einen Schritt des Bereitstellens einer ersten Transferdruckfolie und einer zweiten Transferdruckfolie aufweisen, wobei die Schicht aus elektrisch leitfähigem Polymer und die Schicht aus elektrisch leitfähigem Gewebe zwischen der ersten Transferdruckfolie und der zweiten Transferdruckfolie angeordnet sind, wobei die erste Transferdruckfolie auf der Schicht aus elektrisch leitfähigem Polymer angeordnet ist, wobei die erste Transferdruckfolie in einem zu der Schicht aus elektrisch leitfähigem Polymer benachbarten Bereich teilweise geöffnet ist, so dass die Schicht aus elektrisch leitfähigem Polymer teilweise freiliegt.

Bei Ausführungsbeispielen können die die Schicht aus elektrisch leitfähigem Polymer und die Schicht aus elektrisch leitfähigem Gewebe mittels des Transferdruckverfahrens zwischen der ersten Transferdruckfolie und der zweiten Transferdruckfolie eingebettet werden.

Bei Ausführungsbeispielen können bei dem Bilden der zumindest zwei Trockenelektroden ferner zumindest zwei isolierte Leitungen gebildet werden.

Bei Ausführungsbeispielen kann das Verfahren ferner einen Schritt des Bildens eines Anschlusses [z.B. zum Bereitstellen der mit den zumindest zwei Trockenelektroden erfassten elektrophysiologischen Signale] an einer Außenseite des textilen Trägers aufweisen, wobei der Anschluss mit den zumindest zwei isolierten Leitungen verbunden ist.

Bei Ausführungsbeispielen können bei dem Bilden des Anschlusses nach außen geführte Anschlusselemente [z.B. Druckknöpfe oder Bananenbuchsen] gebildet werden, die mit jeweils einer der zumindest zwei isolierten Leitungen verbunden sind.

Bei Ausführungsbeispielen kann bei dem Bilden des Anschlusses eine weitere Transferdruckfolie bereitgestellt werden, wobei die weitere Transferdruckfolie in Bereichen der nach außen geführten Anschlusselementen geöffnet ist, so dass die Anschlusselemente freiliegen, wobei die weitere Transferdruckfolie mittels eines Transferdruckverfahrens an der Außenseite des textilen Trägers angebracht wird, um den Anschluss zu bilden.

Weitere Ausführungsbeispiele schaffen ein Verfahren zur Erfassung eines mehrkanaligen Elektrokardiogramms eines Lebewesens mit einer anziehbaren Vorrichtung mit zumindest vier Trockenelektroden gem. einem der hierin beschriebenen Ausführungsbeispiele. Das Verfahren umfasst einen Schritt des Einkoppelns eines Referenzsignals in das Lebewesen mit einer Trockenelektrode der zumindest vier Trockenelektroden der anziehbaren Vorrichtung. Das Verfahren umfasst ferner einen Schritt des Erfassens von zumindest drei elektrophysiologischen Signalen des Lebewesens mittels zumindest drei anderen Trockenelektroden der zumindest vier Trockenelektroden der anziehbaren Vorrichtung, um das mehrkanalige Elektrokardiogramm zu erhalten [z.B. mittels Verarbeiten der erfassten zumindest drei elektrophysiologischen Signalen].

Hierin beschriebene Ausführungsbeispiele schaffen einen textilen Träger, mit dem sowohl die oben beschriebenen Zielsetzungen hinsichtlich stabiler, komfortabler Signalerfassung physiologischer Parameter im Alltag und mobilen Einsatz umsetzen lassen, dabei aber ohne die Nachteile bisheriger Technologien realisiert werden können.

Ein erster Aspekt bezieht sich auf einen textilen Träger mit einem einstellbares Gurtsystem, das ähnlich wie eine Weste angezogen bzw. ein Rucksack aufgesetzt werden kann. Bei Ausführungsbeispielen lassen sich die Gurte z.B. über ein System aus Klettverschlüssen an beiden Schultern sowie am Bauch jederzeit auf die Körpergröße und Körperproportionen der Anwenderin oder des Anwenders anpassen. Durch die Anpassung des Gurtsystems wird vor allem die Adaption der primären Messwertaufnehmer (EKG-Elektroden) beeinflusst, sodass immer ein ausreichend guter Haut-Elektroden-Kontakt und damit eine hohe Signalqualität. Die Einstellung muss nicht vor dem Anlegen des textilen Trägers erfolgen (wie z.B. die Einstellschnalle bei einem Pulsbrustgurt zur initialen Weitenänderung), sondern kann auch in der Phase der Datenerfassung immer wieder auf die Aktivitäten und Bedürfnisse des Trägers angepasst werden. Aufgrund des Schnittmusters (keine Stoffpartien vorne am Brustkorb) ist das Trägertextil implizit für Frauen und Männer geeignet. Sowohl die Einstellbarkeit auf die aktuelle Situation und Tätigkeit als auch die Konzeption als geschlechterunabhängiges Textil, unterstützen die Zielerreichung einer kontinuierlich guten Adaption und damit einer hohen Signalqualität bei der Signalerfassung im Alltag (auch über verschiedene Phasen Im tagesverlauf hinweg).

Ein zweiter Aspekt, welcher die Zielsetzung einer guten Signalqualität unterstützt, bezieht sich auf eine geschickte Auswahl und Kombination von jeweils den am besten geeigneten Materialien für die Signalerfassung, Signalübertragung und Störunterdrückung. Die Übertragungsstrecke von den primären Messwertaufnehmern hin zur Kontaktierungsstelle für die Auswerteelektronik gliedert sind in folgende Funktionsblöcke:
▪ Elektrodenmaterial, das in direktem Kontakt mit der Hautoberfläche steht und die physiologischen Parameter aufnimmt. Die Elektroden sollen hohen Leitwert, einen ausgeprägten Hafteffekt auf der Hautoberfläche (kein Wegrutschten bei Bewegungen) und Biokompatibilität aufweisen.
▪ Die Messleitung soll die aufgenommenen Signale verlustfrei und störungsarm übertragen. Das bedeutet, dass das verwendete Material und die aufgebaute Struktur einen hohen Leitwert, eine ausreichen Dehnbarkeit und geringe Widerstandänderung bei mechanischer Beeinflussung aufweisen muss.
▪ Die Isolationsschicht schützt das erfasste Signal in der Messleitung vor Störeinkopplungen.

Für alle Komponenten gilt, dass die geforderten Eigenschaften durch die chemischen und mechanischen Einflüsse beim Waschen nicht verändert werden dürfen.

Ein dritter Aspekt zur Verbesserung der Qualität erfasster Signale bezieht sich auf die Art und Weise der Integration der ausgewählten Materialien in das textile Trägersystem. Bei Ausführungsbeispielen werden Elektrodenmaterial, Messleitung und Isolationsschicht in einem mehrschichtigen Strukturaufbau eingebracht:
▪ Die unterste Schicht direkt auf dem Trägertextil ist zum einen untere Isolationsebene und zugleich die Trägerfläche für das gesamte System aus Messwertaufnehmer und Messleitung (unterer Mantel).
▪ Die zweite Schicht ist ein leitfähiges Material (Kern).
▪ Die dritte Schicht ist ein elektrisch leitfähiges und hautfreundliches Silikon, das nur lokal bei der Elektrodenfläche platziert wird. Damit das darunterliegende leitfähige Material vor Auswaschung geschützt und zugleich eine gute Signalübertragung von Haut zu Silikon zu Messleitung gebildet wird (Elektrode).
▪ Die vierte Schicht ist die obere Isolationsschicht, die bis auf die Elektrodenfläche (bereits durch die dritte Schicht abgedeckt) alle Bereiche der noch offenen Messleitung abdeckt (oberer Mantel).

Die aus den beiden Mantelflächen aufgebaute Struktur erfüllt im textilen Trägersystem folgende drei Aufgaben:
▪ Elektrische Isolation und Schutz der erfassten Signale und damit Vermeidung von Störsignalen und Kurzschlüssen.
▪ Mechanische Stabilisierung der Messleitung und Reduzierung von Bewegungsartefakten.
▪ Schutz des leitfähigen Materials vor Auswaschen und damit Erhaltung der physikalischen Materialeigenschaften.

Zur erweiterten Dehnbarkeit der Messleitungen bei Bewegungen des Anwenders, können diese in einer Mäanderstruktur ausgeführt sein. Beim Dehnen und Stauchen findet dann eine geringere mechanische Belastung und damit eine geringe Widerstandsmodulation statt.

Ausführungsbeispiele schaffen einen textilen Träger, der die oben beschriebenen Merkmale in der folgenden Art und Weise zusammenführt:
▪ Kombination geeigneter Werkstoffe für ein Sensorsystem mit Trockenelektroden mit hohem Tragekomfort und verbesserter (z.B. bestmöglicher) Signalqualität.
▪ Konzeption eines einstellbaren textilen Trägers für flexible und verbesserter (z.B. guter Adaption).
▪ Integration der Funktionskomponenten für verbesserte (z.B. höchste) Störstabilität und geringer Artefaktanfälligkeit.
▪ Ergänzend weist das entwickelte Trägersystem eine einfache Handhabung, flexible Anpassung, Unauffälligkeit und hohen Tragekomfort auf, so dass von einer hohen Nutzerakzeptanz und damit sowohl anwendungsbezogenen Verbesserungen als auch kommerziellen Erfolgen zu rechnen ist.

Bei Ausführungsbeispielen erzeugen die verwendeten Materialien eine Signalübertragungskette, mit der verbesserte (z.B. bestmögliche) Signalqualitäten erzielt werden können.

Bei Ausführungsbeispielen ermöglicht das Konzept des Trägertextils eine individuelle und zeitliche flexible Anpassbarkeit, so dass auch bei unterschiedlichen Personen und Aktivitäten immer eine ausreichend gute Adaption und damit eine gute Signalerfassung gewährleistet ist. Bei Ausführungsbeispielen erzeugt die Art und Weise der Integration eine verbesserte (z.B. gute) Störstabilität und Artefaktstabilität für die Messleitung, Langzeitstabilität über längere Verwendungsdauer beim Monitoring im Alltag (insbesondere hinsichtlich Waschzyklen).

Ausführungsbeispiele der vorliegenden Erfindung finden Anwendung beim medizinischen Monitoring von Patienten mit kardiovaskulärer Risikokonstellation, beim medizinischen Monitoring von Patienten in einer kardiologischen Rehabilitation und beim telemedizinischen Kardio-Monitoring.

Ausführungsbeispiele der vorliegenden Erfindung werden bezugnehmend auf die beiliegenden Figuren näher beschrieben. Es zeigen:
- Fig. 1a: eine schematische Vorderansicht einer Vorrichtung zur Erfassung elektrophysiologischer Signale eines Lebewesens, gemäß einem Ausführungsbeispiel der vorliegenden Erfindung,
- Fig. 1b: eine schematische Rückansicht der Vorrichtung zur Erfassung elektrophysiologischer Signale eines Lebewesens, gemäß einem Ausführungsbeispiel der vorliegenden Erfindung,
- Fig. 2a: eine Illustration einer Vorderansicht der Vorrichtung zur Erfassung elektrophysiologischer Signale eines Lebewesens, wenn diese beispielhaft von einer Puppe als Repräsentation für ein Lebewesen getragen wird,
- Fig. 2b: eine Illustration einer Rückansicht der Vorrichtung zur Erfassung elektrophysiologischer Signale eines Lebewesens, wenn diese beispielhaft von einer Puppe als Repräsentation für ein Lebewesen getragen wird,
- Fig. 3: eine schematische Ansicht einer Trockenelektrode, gemäß einem Ausführungsbeispiel der vorliegenden Erfindung,
- Fig. 4: ein Flussdiagramm eines Verfahrens zur Herstellung einer Vorrichtung zur Erfassung elektrophysiologischer Signale, gemäß einem Ausführungsbeispiel der vorliegenden Erfindung,

- Fig. 5: eine schematische Ansicht eines Schichtaufbaus einer Trockenelektrode sowie eines Abschnitts einer Leitung, gemäß einem Ausführungsbeispiel der vorliegenden Erfindung,
- Fig. 6: eine schematische Ansicht einer Innenseite der Vorrichtung zur Erfassung elektrophysiologischer Signale, gemäß einem Ausführungsbeispiel der vorliegenden Erfindung,
- Fig. 7: eine schematische Detailansicht eines außenliegenden Teils des Anschlusses mit den Verbindungselementen, gemäß einem Ausführungsbeispiel der vorliegenden Erfindung, und
- Fig. 8: eine schematische Detailansicht eines innenliegenden Teils des Anschlusses, der die Verbindungen zwischen den isolierten Leitungen und den an der Außenseite des textilen Trägers frei liegenden Verbindungselementen realisiert.

In der nachfolgenden Beschreibung der Ausführungsbeispiele der vorliegenden Erfindung werden in den Figuren gleiche oder gleichwirkende Elemente mit dem gleichen Bezugszeichen versehen, so dass deren Beschreibung untereinander austauschbar ist.

### Vorrichtung (Gurtsystem) zur Erfassung elektrophysiologischer Signale

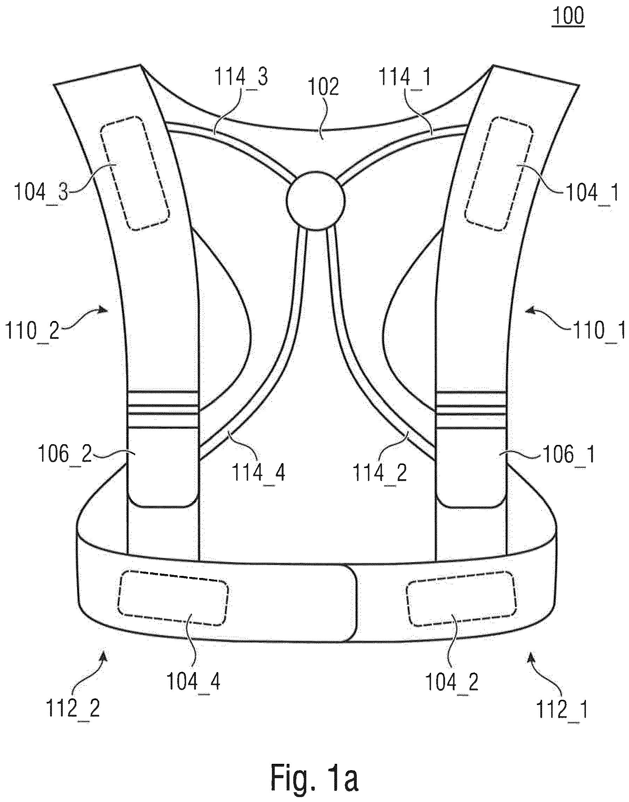

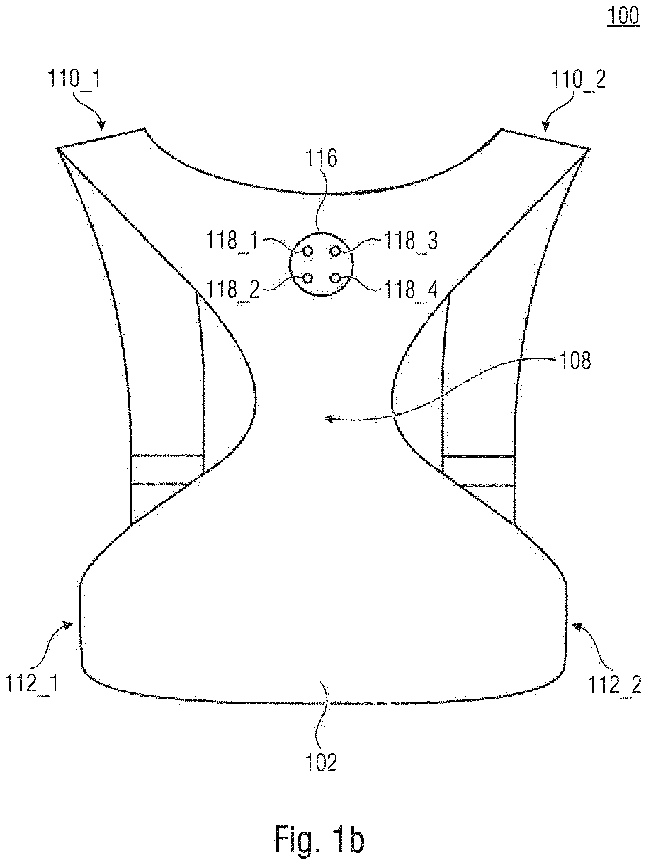

Fig. 1a zeigt eine schematische Vorderansicht einer Vorrichtung 100 zur Erfassung elektrophysiologischer Signale eines Lebewesens (z.B. Tieres oder Menschen) und Fig. 1b eine schematische Rückansicht der Vorrichtung 100 zur Erfassung elektrophysiologischer Signale eines Lebewesens, gemäß einem Ausführungsbeispiel der vorliegenden Erfindung.

Die Vorrichtung 100 umfasst einen textilen Träger 102, zumindest zwei Trockenelektroden 104_1-104_4, die auf einer Innenseite des textilen Trägers 102 angebracht sind, und zumindest zwei an dem textilen Träger 102 angebrachten Gurten 106_1-106_2. Die zumindest zwei Gurte 106_1-106_2 ermöglichen es hierbei die anziehbare Vorrichtung 100 auf einen Körper des Lebewesens und/oder einen Anpressdruck der zumindest zwei Trockenelektroden 104 auf eine Haut des Lebewesens einzustellen, z.B. wodurch eine Qualität der durch die zumindest zwei Trockenelektroden 104_1-104_4 erfassbaren physiologischen Signale verbessert werden kann.

In den Fig. 1a und 1b wird beispielhaft davon ausgegangen, dass die Vorrichtung 100 vier Trockenelektroden aufweist. Es sei jedoch darauf hingewiesen, dass die Erfindung nicht auf solche Ausführungsbeispiele beschränkt ist. Vielmehr kann die Vorrichtung 100 im Allgemeinen n Trockenelektroden 104_1-104_n aufweisen, wobei n eine natürliche Zahl größer gleich zwei ist, n ≥ 2, wie zum Beispiel zwei, drei, vier, fünf, sechs, oder mehr Trockenelektroden .

Wie dies in den Fig. 1a und 1b beispielhaft gezeigt ist, kann der textile Träger 102 aus einem (z.B. einzigen) Materialstück gefertigt sein und derart geformt sein, dass dieser fünf Bereiche aufweist: Einen mittleren Bereich 108, zwei obere Bereiche 110_1-110_2, die sich jeweils von dem mittleren Bereich 108 weg erstrecken, und zwei seitliche Bereiche 112_1 -112_2, die sich jeweils von dem mittleren Bereich 108 weg erstrecken, wobei, wenn die Vorrichtung 100 von einem Lebewesen getragen wird, sich der mittlere Bereich 108 des textilen Trägers 102 über einen Rückenbereich des Lebewesen erstreckt, sich die zwei oberen Bereiche 110_1-110_2 des textilen Trägers 102 ausgehend von dem Rückenbereich über jeweilige Schulterbereiche bis hin zu oberen Brustbereichen des Lebewesen erstrecken, und sich die zwei seitlichen Bereiche 112_1-112_2 des textilen Trägers 102 ausgehend von dem Rückenbereich über jeweilige Axillarlinien bis hin zu jeweiligen oberen Bauchbereichen oder unteren Brustbereichen erstrecken.

Die zwei oberen Bereiche 110_1-110_2 des textilen Trägers 102 können mit den zwei seitlichen Bereichen 112_1-112_2 des textilen Trägers über zwei der zumindest zwei Gurte verbindbar sind. Beispielsweise kann ein erster oberer Bereich 110_1 des textilen Trägers 102 über einen ersten Gurt 106_1 mit einem ersten unteren Bereich 112_1 des textilen Trägers 102 verbunden sein, während ein zweiter oberer Bereich 110_2 des textilen Trägers 102 über einen zweiten Gurt 106_2 mit einem zweiten unteren Bereich 112_2 des textilen Trägers 102 verbunden sein kann.

Die zwei seitlichen Bereiche 112_1-112_2 können über einen dritten Gurt oder alternativ über ein Verbindungselement, wie z.B. Klettverschluss, Steckschnalle, Klemmriemen oder Druckknopf, miteinander verbindbar sein.

Bei Ausführungsbeispielen können die zumindest zwei Trockenelektroden an zumindest zwei Bereichen aus den zwei oberen Bereichen 110_1-110_2 und den zwei seitlichen Bereichen 112_1-112_2 des textilen Trägers 102 an einer Innenseite des textilen Trägers 102 angebracht sein. Beispielsweise kann, wie dies in den Fig. 1a und 1b gezeigt ist, eine erste Trockenelektrode 104_1 an einem ersten oberen Bereich 110_1 des textilen Trägers 102, eine zweite Trockenelektrode 104_2 an einem ersten unteren Bereich 112_1 des textilen Trägers 102, eine dritte Trockenelektrode 104_3 an einem zweiten oberen Bereich des textilen Trägers 102 und eine vierte Trockenelektrode 104_4 an dem zweiten unteren Bereich 112_2 des textilen Trägers 102 angebracht sein.

Bei Ausführungsbeispielen können die zumindest zwei Trockenelektroden 104_1-104_4 über isolierte Leitungen 114_1-114_4 mit einem an einer Außenseite des textilen Trägers 102 angebrachten Anschluss 116 verbunden sein, z.B. zum Bereitstellen der mit den zumindest zwei Trockenelektroden erfassten elektrophysiologischen Signale. Hierzu kann der Anschluss 116 nach außen geführte Anschlusselemente 118_1-118_4 aufweisen, die mit den zumindest zwei isolierten Leitungen verbunden sind.

Wie in den Fig. 1a und 1b gezeigt ist, kann der textile Träger 102 zusammen mit den zumindest zwei Gurten 106_1-106_2 beispielsweise die Form eines Gurtsystems bilden. Beim Einsatz des Gurtsystems zur Erfassung physiologeischer Signale am Menschen oder an einem menschähnlichen Tier (z.B. Affen) kann das Gurtsystem beispielsweise die Form einer Weste aufweisen.

Bei Ausführungsbeispielen kann die in den Fig. 1a und 1b gezeigte Vorrichtung 100 z.B. zur Erfassung eines Elektrokardiogramms (EKGs) oder zum Überwachen von Patienten mit kardiovaskulärer Risikokonstellation oder zum Überwachen von Patienten in einer kardiologischen Rehabilitation oder zur telemedizinischen Kardioüberwachung verwendet werden.

Mit anderen Worten, Fig. 1a zeigt eine Vorderansicht der Vorrichtung 100 (Gurtsystem) und Fig. 1b eine Rückansicht der Vorrichtung 100. Der textile Träger 102 (z.B. in Form einer Weste) besteht aus einem einzigen dehnbaren Materialstück, an das die Gurte 106_1-106_2 angenäht sind. Die Gurte 106_1-106_2 sind elastisch und haben Klettverschluss, so dass beim Anlegen eine Enge und ein gleichmäßiger Anpressdruck der Elektroden 104_1-104_4 angepasst werden kann.

Die Elektroden 104_1-104_4 befinden sich auf der Innenseite des Gurtsystems. Damit die Elektroden 104_1-104_4 besser mit Haupt haften haben die Elektroden die rutschfesten Oberflächen. An der Außenseite des Gurtsystems befinden sich auf der Rückseite Kontakte 118_1-118_4 zum Anschluss eines Messgerätes (siehe Fig. 1b).

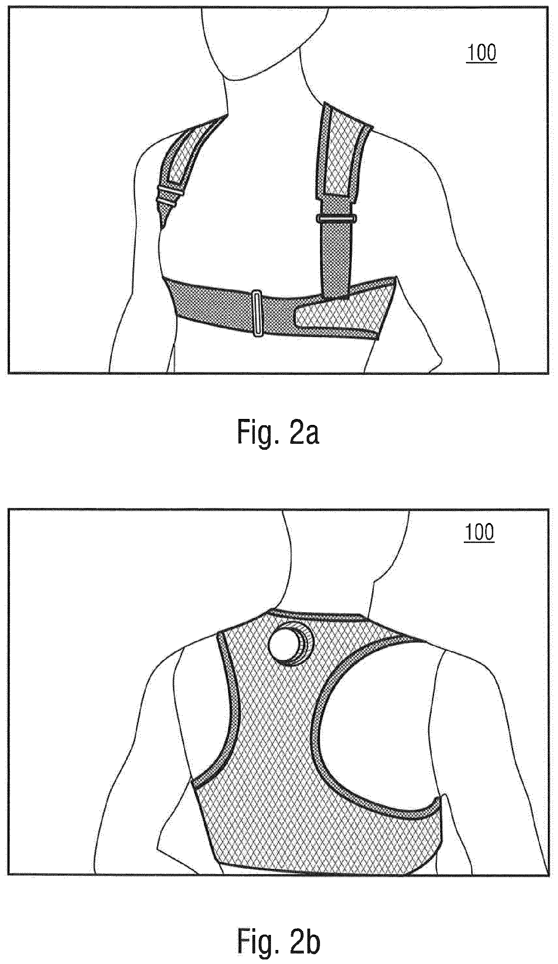

Fig. 2a zeigt eine Illustration einer Vorderansicht der Vorrichtung 100, wenn diese beispielhaft von einer Puppe als Repräsentation für ein Lebewesen getragen wird, während Fig. 2b eine Illustration einer Rückansicht der Vorrichtung 100 zeigt, wenn diese beispielhaft von einer Puppe als Repräsentation für ein Lebewesen getragen wird.

### Trockenelektroden und Kombination von Elektrodenmaterialien

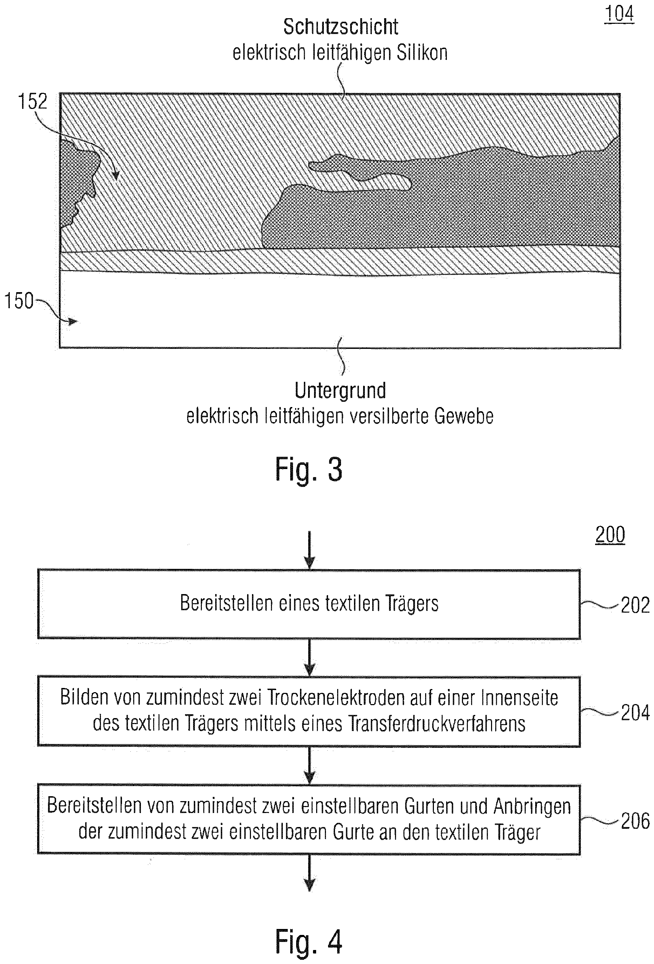

Fig. 3 zeigt eine schematische Ansicht einer Trockenelektrode 104, gemäß einem Ausführungsbeispiel der vorliegenden Erfindung. Wie in Fig. 3 zu erkennen ist, weist die Trockenelektrode 104 eine Schicht 150 aus elektrisch leitfähigem Gewebe und eine Schicht 152 aus elektrisch leitfähigem Polymer, die die Schicht 150 aus leitfähigem Gewebe bedeckt, auf.

Die Anforderungen an den Trockenelektroden 104 bei der EKG-Messung ist, dass der Widerstand der Trockenelektroden 104 niedrig ist und über die Zeit nahezu Konstant bleibt. Außerdem sollten die Trockenelektroden eine gute Haftung auf der Haut des Patienten aufweisen. Um diese Anforderungen zu erfüllen kann bei Ausführungsbeispielen eine Kombination von Materialien zum Einsatz kommen, wie dies im Folgenden erläutert wird.

Bei Ausführungsbeispielen kann als elektrisch leitfähiger Stoff ein versilbertes Strickgewebe als Grundmaterial zum Einsatz kommen. Der Vorteil dieses Stoffs ist seine verbesserte (z.B. hohe) elektrische Leitfähigkeit, aber beim Waschen in offenen Bereichen den Elektroden werden Silberpartikel ausgewaschen. Dies führt zu einer Erhöhung des elektrischen Widerstands, was eine Verschlechterung des EKG-Signals zur Folge hat. Um das Auswaschen von Silberpartikeln zu vermeiden, wird dieses Gewebe bei Ausführungsbeispielen mit einem elektrisch leitfähigen Polymer, wie z.B. einer Silikonbeschichtung, geschützt (siehe Fig. 3). Elektrisch leitfähiges Silikon hat eine niedrigere Leitfähigkeit als das versilberte Strickgewebe, aber wenn dieses in einer dünnen Schicht (z.B. 1 mm oder weniger) auf die Oberfläche des versilberten Strickgewebes aufgetragen wird, wird die verbesserte (z.B. hohe) Leitfähigkeit der Trockenelektrode 104 beibehalten und gleichzeitig die Trockenelektrode 104 vor dem Auswaschen von Silberpartikeln geschützt. Darüber hinaus hat Silikon aufgrund seiner guten Elastizität eine gute Hafteigenschaft auf der Haut eines Lebewesens (z.B. Menschen oder Tieres). Diese Materialkombination ermöglicht es, die Trockenelektroden mehrfach zu verwenden und diese in waschbarer Kleidung zu benutzen.

### Integration von Materialien

Fig. 4 zeigt ein Flussdiagramm eines Verfahrens 200 zur Herstellung einer Vorrichtung 100 zur Erfassung elektrophysiologischer Signale, gemäß einem Ausführungsbeispiel der vorliegenden Erfindung. Das Verfahren 200 umfasst einen Schritt 202 des Bereitstellens eines textilen Trägers. Ferner umfasst das Verfahren 200 einen Schritt 204 des Bildens von zumindest zwei Trockenelektroden auf einer Innenseite des textilen Trägers mittels eines Transferdruckverfahrens. Ferner umfasst das Verfahren 200 einen Schritt 206 des Bereitstellens von zumindest zwei einstellbaren Gurten und Anbringen der zumindest zwei einstellbaren Gurte an den textilen Träger.

Bei Ausführungsbeispielen können bei dem Schritt 204 des Bildens der zumindest zwei Trockenelektroden auch die mit den zumindest zwei Trockenelektroden verbundenen isolierten Leitungen und (optional) auch der Anschluss, der mit den isolierten Leitungen verbunden ist, gebildet werden.

Im Folgenden werden bevorzugte Ausführungsbeispiele des Schritts 204 näher beschrieben.

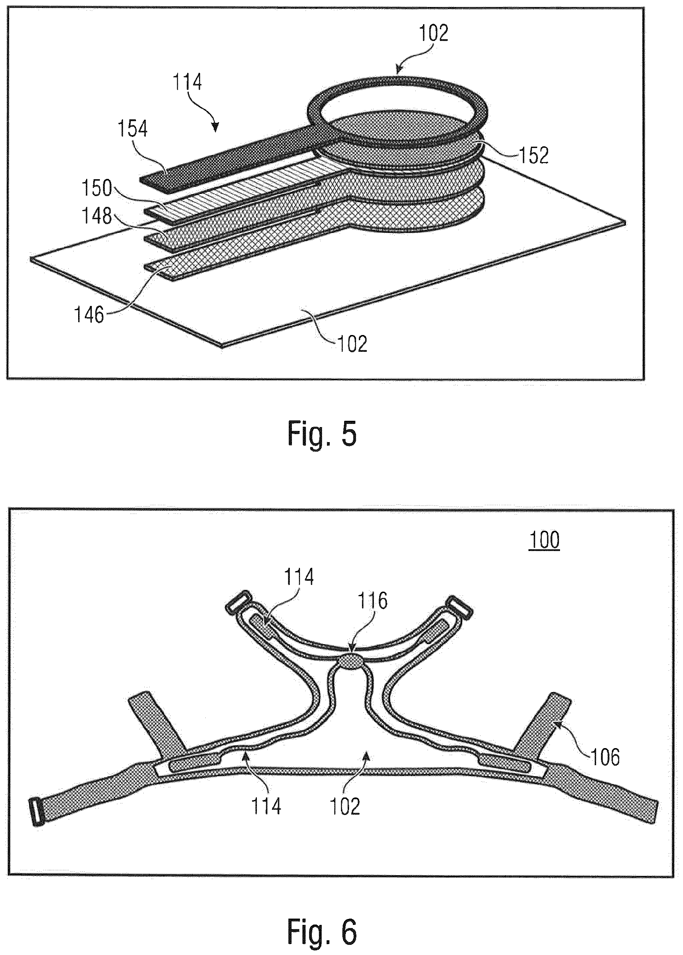

Um die Vorrichtung 100 alltagstauglich und widerstandsfähig gegen wiederverwendbare Wäsche zu machen, kann bei Ausführungsbeispielen das Transferdruckverfahren verwendet werden. Normalerweise wird das Transferdruckverfahren zum Dekorieren und Abdichten von Kleidung verwendet. Bei Ausführungsbeispielen wird dieses Verfahren verwendet, um alle Komponenten (Trockenelektroden, Leitungen, Anschluss) mit den Grundtextilien (textilen Träger) zu verschweißen und elektrisch leitende Materialien abzudichten. Zu diesem Zweck kann ein mehrschichtiger Materialaufbau verwendet werden, wie dies im Folgenden anhand von Fig. 5 erläutert wird.

Im Detail zeigt Fig. 5 eine schematische Ansicht eines Schichtaufbaus (z.B. mehrschichtige Integrationsstruktur) einer Trockenelektrode 104 sowie eines Abschnitts einer Leitung 114, gemäß einem Ausführungsbeispiel der vorliegenden Erfindung.

In einer Vorbereitungsphase können alle Schichten für die Trockenelektrode 104 und die isolierte Leitung 114 bereitgestellt werden, z.B. nach einer Schablone ausgeschnitten und dann übereinander gelagert werden. Wie in Fig. 5 zu erkennen ist, können die Schichten in der folgenden Reihenfolge (ausgehend von dem textilen Träger 102) angebracht werden:
▪ textiler Träger 102,
▪ Transferdruckfolie 146,
▪ thermoplastische Polyurethanfolie (TPU) 148,
▪ leitfähiges Gewebe 150,
▪ leitfähige Polymerschicht (z.B. Silikonschicht) 152 (nur auf dem leitfähigen Gewebe 150 im Bereich der Trockenelektrode 102), und
▪ Transferdruckfolie 154 (geöffnet im Bereich der Trockenelektrode 102, so dass die leitfähige Polymerschicht 152 teilweise freiliegt (z.B. nur an am Rand von der Transferdruckfolie 154 bedeckt ist)).

Nach dem die obigen Schichten verlegt sind, kann dieser Schichtaufbau (Sandwichstruktur) unter Druck erhitzt werden (z.B. 170°C). Das Ergebnis der Verklebung ist in den Fig. 6 bis 8 illustriert.

Hierbei zeigt Fig. 6 eine schematische Ansicht einer Innenseite der Vorrichtung 100, gemäß einem Ausführungsbeispiel der vorliegenden Erfindung. Wie in Fig. 6 zu erkennen ist, sind an einer Innenseite des textilen Trägers 102 die Trockenelektroden 104, isolierten Leitungen 114 und ein innenliegender Teil des Anschlusses 116 angebracht. Der innenliegende Teil des Anschlusses 116 kann Verbindungen zwischen den isolierten Leitungen 114 und den an der Außenseite des textilen Trägers frei liegenden Verbindungselementen realisieren. In Fig. 6 sind ferner die an dem textilen Träger angebrachten Gurte 106 zu erkennen.

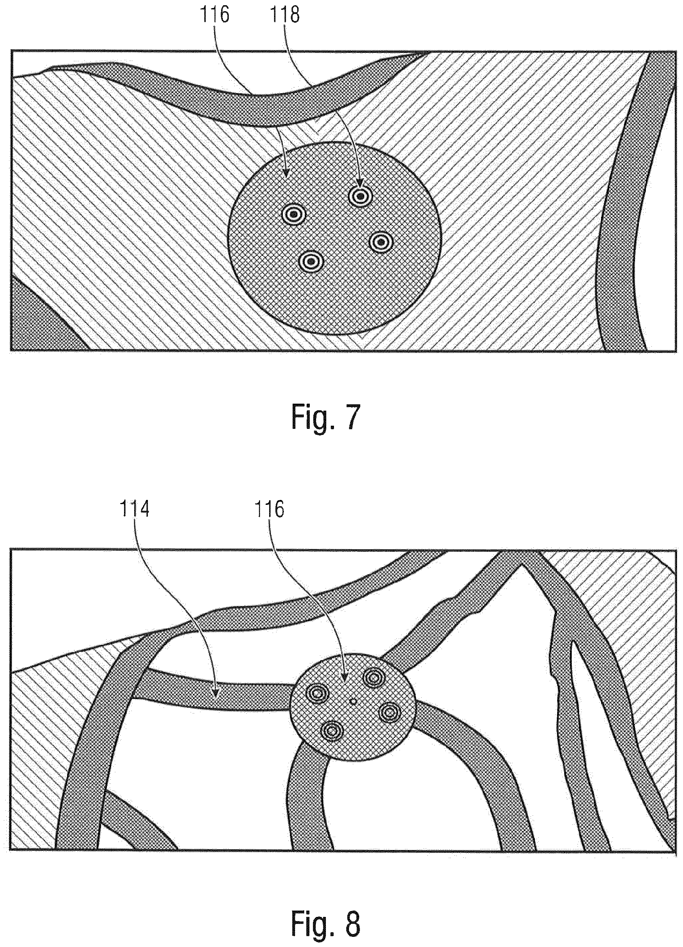

Fig. 7 zeigt eine schematische Detailansicht eines außenliegenden Teils des Anschlusses 116 mit den Verbindungselementen 118, gemäß einem Ausführungsbeispiel der vorliegenden Erfindung. Die Verbindungselemente können z.B. mittels Druckknöpfen oder Bananenbuchsen realisiert werden.

Fig. 8 zeigt eine schematische Detailansicht eines innenliegenden Teils des Anschlusses 116, der die Verbindungen zwischen den isolierten Leitungen 114 und den an der Außenseite des textilen Trägers frei liegenden Verbindungselementen realisiert.

Der oben beschriebene Aufbau von Leitungen ist wasserdicht und schützt vor Feuchtigkeit oder Schweiß während der Nutzung.

### Weitere Ausführungsbeispiele

Zur Aufnahme von physiologischen Signalen in möglichst hoher Qualität sowie zur Vermeidung von Störeinflüssen bei der Datenerfassung (insbesondere in Anwendungsszenarien im mobilen Alltag) stellt die ausreichend gute und zuverlässige Adaption der primären Messwertaufnehmer (hier EGK-Elektroden) an den menschlichen Körper eine große Herausforderung dar. Ausführungsbeispiele realisieren erstens eine Kombination verbesserter (z.B. bestmöglicher) Materialkomponenten für Signalerfassung und Signalübertragung. Zweitens setzen Ausführungsbeispiele die gewählte Form der Integration dieser Materialien in einen textilen Träger die Anforderungen nach Störstabilität um. Drittens adressieren und entschärfen Ausführungsbeispiele durch das gewählte Konzept (als einstellbares Gurtsystem) weitere kritische Punkte, so dass für verschiedene Geschlechter, Körpergrößen und Körperproportionen immer eine ausreichend gute Signalqualität bereitgestellt werden kann.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Anziehbare Vorrichtung (100) zur Erfassung elektrophysiologischer Signale eines Lebewesens, mit folgenden Merkmalen:
einem textilen Träger (102),
zumindest zwei Trockenelektroden (104_1-104_4), die auf einer Innenseite des textilen Trägers (102) angebracht sind, und
zumindest zwei an dem textilen Träger (102) angebrachten, einstellbaren Gurten (106_1-106_2), die es ermöglichen, die anziehbare Vorrichtung (100) auf einen Körper des Lebewesens und einen Anpressdruck der zumindest zwei Trockenelektroden (104_1-104_4) auf eine Haut des Lebewesens einzustellen.

2. Anziehbare Vorrichtung (100) nach dem vorangehenden Anspruch,
wobei die zumindest zwei Trockenelektroden (104_1-104_4) zumindest vier Trockenelektroden (104_1-104_4) zur Erfassung eines mehrkanaligen Elektrokardiogramms des Lebewesens sind.

3. Anziehbare Vorrichtung (100) nach einem vorangehenden Ansprüche,
wobei der textile Träger (102) aus einem Materialstück gefertigt ist.

4. Anziehbare Vorrichtung (100) nach einem der vorangehenden Ansprüche,
wobei der textile Träger (102) zusammen mit den zumindest zwei Gurten (106_1-106_2) die Form eines Gurtsystems bilden.

5. Anziehbare Vorrichtung (100) nach einem der vorangehenden Ansprüche,
wobei der textile Träger (102) einen mittleren Bereich (108), zwei obere Bereiche (110_1-110_2), die sich jeweils von dem mittleren Bereich (108) weg erstrecken, und zwei seitliche Bereiche (112_1-112_2), die sich jeweils von dem mittleren Bereich (108) weg erstrecken, aufweist.

6. Anziehbare Vorrichtung (100) nach Anspruch 5,
wobei, wenn die Vorrichtung (100) von dem Lebewesen getragen wird, sich der mittlere Bereich (108) des textilen Trägers (102) über einen Rückenbereich des Lebewesen erstreckt, sich die zwei oberen Bereiche (110_1-110_2) des textilen Trägers (102) ausgehend von dem Rückenbereich über jeweilige Schulterbereiche bis hin zu oberen Brustbereichen des Lebewesen erstrecken, und sich die zwei seitlichen Bereiche (112_1-112_2) des textilen Trägers (102) ausgehend von dem Rückenbereich über jeweilige Axillarlinien bis hin zu jeweiligen oberen Bauchbereichen oder unteren Brustbereichen erstrecken.

7. Anziehbare Vorrichtung (100) nach einem der Ansprüche 5 bis 6,
wobei die zwei oberen Bereiche (110_1-110_2) des textilen Trägers (102) mit den zwei seitlichen Bereichen (112_1 -112_2) des textilen Trägers (102) über zwei der zumindest zwei Gurte (106_1-106_2) verbindbar sind.

8. Anziehbare Vorrichtung (100) nach einem der Ansprüche 5 bis 7,
wobei die zwei seitlichen Bereiche (112_1-112_2) des textilen Trägers (102) über ein Verbindungselement miteinander verbindbar sind,
oder wobei die zwei seitlichen Bereiche (112_1-112_2) des textilen Trägers (102) über einen dritten Gurt der zumindest zwei Gurte (106_1-106_2) miteinander verbindbar sind.

9. Anziehbare Vorrichtung (100) nach einem der Ansprüche 5 bis 8,
wobei die zumindest zwei Trockenelektroden (104_1-104_4) an zumindest zwei Bereichen aus den zwei oberen Bereichen (110_1-110_2) und den zwei seitlichen Bereichen (112_1-112_2) des textilen Trägers (102) an dem textilen Träger (102) angebracht sind.

10. Anziehbare Vorrichtung (100) nach einem der Ansprüche 5 bis 9,
wobei die zumindest zwei Trockenelektroden (104_1-104_4) zumindest vier Trockenelektroden (104_1-104_4) sind,
wobei zwei Trockenelektroden (104_1, 104_3) der zumindest vier Trockenelektroden auf einer Innenseite der zwei oberen Bereiche (110_1-110_2) des textilen Trägers (102) angebracht sind,
wobei zwei andere Trockenelektroden (104_2, 104_4) der zumindest vier Trockenelektroden auf einer Innenseite der zwei seitlichen Bereiche (112_1-112_2) des textilen Trägers (102) angebracht sind.

11. Anziehbare Vorrichtung (100) nach Anspruch 10,
wobei, wenn die Vorrichtung (100) von dem Lebewesen getragen wird, die zwei Trockenelektroden (104_1, 104_3) Schlüsselbeinbereiche [z.B. linken und rechten Schlüsselbeinbereich] oder obere Bereiche oberhalb eines Brustbereich des Lebewesens kontaktieren,
und/oder wobei, wenn die Vorrichtung (100) von dem Lebewesen getragen wird, die zwei anderen Trockenelektroden (104_1, 104_4) abdominale Bereiche [z.B. innerhalb eines abdominalen Quadranten] oder untere Bereiche unterhalb eines Brustbereichs des Lebewesens kontaktieren.

12. Anziehbare Vorrichtung (100) nach einem der vorangehenden Ansprüche,
wobei der textile Träger (102) und/oder die zumindest zwei Gurte (106_1-106_2) elastisch sind.

13. Anziehbare Vorrichtung (100) nach einem der vorangehenden Ansprüche,
wobei die zumindest zwei Trockenelektroden (104_1-104_4) jeweils eine Schicht (150) aus leitfähigem Gewebe und eine Schicht (152) aus elektrisch leitfähigem Polymer, die die Schicht (150) aus leitfähigem Gewebe bedeckt, aufweisen.

14. Anziehbare Vorrichtung (100) nach Anspruch 13,
wobei die Schicht (152) aus elektrisch leitfähigem Polymer dünner ist als 1 mm.

15. Anziehbare Vorrichtung (100) nach einem der Ansprüche 13 bis 14,
wobei Schichten (150,152) der zumindest zwei Trockenelektroden (104_1-104_4) mittels einer Kombination aus einem Siebdruckverfahren und einem Transferdruckverfahren gebildet sind.

16. Anziehbare Vorrichtung (100) nach einem der vorangehenden Ansprüche,
wobei die zumindest zwei Trockenelektroden (104_1-104_4) über isolierte Leitungen (114_1-144_4) mit einem an einer Außenseite des textilen Trägers (102) angebrachten Anschluss (116) verbunden sind.

17. Anziehbare Vorrichtung (100) nach Anspruch 16,
wobei die isolierten Leitungen (114_1-114_4)jeweils eine Schicht (150) aus leitfähigem Gewebe und zumindest eine Schicht (154) aus Isolationsmaterial, die die Schicht (150) aus leitfähigem Gewebe bedeckt, aufweisen.

18. Anziehbare Vorrichtung (100) nach Anspruch 17,
wobei Schichten (150, 154) der isolierten Leitungen (114_1-114_4) mittels einer Kombination aus einem Siebdruckverfahren und einem Transferdruckverfahren gebildet sind.

19. Anziehbare Vorrichtung (100) nach einem der Ansprüche 16 bis 18,
wobei die isolierten Leitungen (114_1-114_4) jeweils mit einem nach außen geführten Anschlusselement (118_1-118_4) des Anschlusses (116) verbunden ist.

20. Anziehbare Vorrichtung (100) nach Anspruch 19,
wobei der Anschluss (116) zumindest eine Schicht aus Isolationsmaterial aufweist,
wobei eine Schicht aus der zumindest einen Schicht aus Isolationsmaterial in Bereichen der Anschlusselemente (118_1-118_4) geöffnet ist, so dass die Anschlusselemente frei liegen.

21. Trockenelektrode (114) zur Erfassung elektrophysiologischer Signale eines Lebewesens, mit folgenden Merkmalen:
einer Schicht (150) aus leitfähigem Gewebe, und
einer Schicht (152) aus elektrisch leitfähigem Polymer, die die Schicht (150) aus leitfähigem Gewebe bedeckt.

22. Trockenelektrode (114) nach Anspruch 21,
wobei das leitfähige Gewebe ein versilbertes Gewebe ist.

23. Trockenelektrode (114) nach einem der Ansprüche 21 bis 22,
wobei die Schicht (152) aus elektrisch leitfähigem Polymer eine Dicke von Weniger als 1 mm aufweist.

24. Trockenelektrode (114) nach einem der Ansprüche 21 bis 23,
wobei die Trockenelektrode (114) ferner eine thermoplastische Polyurethanfolie aufweist,
wobei die Schicht (150) aus leitfähigem Gewebe auf der thermoplastischen Polyurethanfolie (148) angeordnet ist.

25. Trockenelektrode (114) nach einem der Ansprüche 21 bis 24,
wobei die Trockenelektrode (114) in einem Transferdruckfolienschichtsystem aus zumindest zwei Transferdruckfolien (146,154) eingebettet ist,
wobei das Transferdruckfolienschichtsystem in einem zu der Schicht (152) aus elektrisch leitfähigem Polymer benachbarten Bereich teilweise geöffnet ist, so dass die Schicht (152) aus elektrisch leitfähigem Polymer teilweise freiliegt.

26. Trockenelektrode (114) nach einem der Ansprüche 21 bis 25,
wobei die Trockenelektrode (114) mittels eines Transferdruckverfahrens auf einem textilen Träger (102) angebracht ist.

27. Verfahren (200) zur Herstellung einer anziehbaren Vorrichtung (100) zur Erfassung elektrophysiologischer Signale eines Lebewesens, wobei das Verfahren (200) aufweist:
Bereitstellen eines textilen Trägers (102),
Bilden von zumindest zwei Trockenelektroden (104_1-104_4) auf einer Innenseite des textilen Trägers (102) mittels eines Transferdruckverfahrens,
Bereitstellen von zumindest zwei einstellbaren Gurten (106_1-106_2) und Anbringen der zumindest zwei einstellbaren Gurte (106_1-106_2) an den textilen Träger (102).

28. Verfahren (200) nach Anspruch 27,
wobei das Bilden der zumindest zwei Trockenelektroden (104_1-104_4) aufweist:
Bereitstellen einer Schicht (150) aus elektrisch leitfähigem Gewebe, und
Bereitstellen einer Schicht (152) aus elektrisch leitfähigem Polymer auf der Schicht (150) aus elektrisch leitfähigem Gewebe, so dass die Schicht (152) aus elektrisch leitfähigem Polymer die Schicht (150) aus elektrisch leitfähigem Gewebe zumindest teilweise bedeckt.

29. Verfahren (200) nach Anspruch 28,
wobei das Bilden der zumindest zwei Trockenelektroden (104_1-104_4) ferner aufweist:
Bereitstellen einer thermoplastischen Polyurethanfolie (148),
wobei die Schicht (150) aus leitfähigem Gewebe auf der thermoplastischen Polyurethanfolie (148) angeordnet ist.

30. Verfahren (200) nach einem der Ansprüche 28 bis 29,
wobei das Bilden der zumindest zwei Trockenelektroden (104_1-104_4) ferner aufweist:
Bereitstellen einer ersten Transferdruckfolie (154) und einer zweiten Transferdruckfolie (146),
wobei die Schicht (152) aus elektrisch leitfähigem Polymer und die Schicht (150) aus elektrisch leitfähigem Gewebe zwischen der ersten Transferdruckfolie (154) und der zweiten Transferdruckfolie (146) angeordnet sind,
wobei die erste Transferdruckfolie (154) auf der Schicht (152) aus elektrisch leitfähigem Polymer angeordnet ist, wobei die erste Transferdruckfolie (154) in einem zu der Schicht (152) aus elektrisch leitfähigem Polymer benachbarten Bereich teilweise geöffnet ist, so dass die Schicht (152) aus elektrisch leitfähigem Polymer teilweise freiliegt.

31. Verfahren (200) nach Anspruch 30,
wobei die die Schicht (152) aus elektrisch leitfähigem Polymer und die Schicht (150) aus elektrisch leitfähigem Gewebe mittels des Transferdruckverfahrens zwischen der ersten Transferdruckfolie (154) und der zweiten Transferdruckfolie (146) eingebettet wird.

32. Verfahren (200) nach einem der Ansprüche 27 bis 31,
wobei bei dem Bilden der zumindest zwei Trockenelektroden (104_1-104_4) ferner zumindest zwei isolierte Leitungen (114_1-114_4) gebildet werden.

33. Verfahren (200) nach Anspruch 32,
wobei das Verfahren (200) ferner aufweist:
Bilden eines Anschlusses (116) an einer Außenseite des textilen Trägers (102), wobei der Anschluss (116) mit den zumindest zwei isolierten Leitungen (114_1-114_4) verbunden ist.

34. Verfahren (200) nach Anspruch 33,
wobei bei dem Bilden des Anschlusses (116) nach außen geführte Anschlusselemente (118_1-118_4) gebildet werden, die mit jeweils einer der zumindest zwei isolierten Leitungen (114_1-114_4) verbunden sind.

35. Verfahren (200) nach Anspruch 34,
wobei bei dem Bilden des Anschlusses (116) eine weitere Transferdruckfolie bereitgestellt wird, wobei die weitere Transferdruckfolie in Bereichen der nach außen geführten Anschlusselementen (118_1-118_4) geöffnet ist, so dass die Anschlusselemente (118_1-118_4) freiliegen, wobei die weitere Transferdruckfolie mittels eines Transferdruckverfahrens an der Außenseite des textilen Trägers (102) angebracht wird, um den Anschluss (116) zu bilden.

36. Verfahren zur Erfassung eines mehrkanaligen Elektrokardiogramms eines Lebewesens mittels einer anziehbaren Vorrichtung nach einem der Ansprüche 2 oder 10 bis 11, wobei das Verfahren aufweist:
Anlegen eines Referenzsignals an das Lebewesen mit einer Trockenelektrode der zumindest vier Trockenelektroden der anziehbaren Vorrichtung,
Erfassen von zumindest drei elektrophysiologischen Signalen des Lebewesens mittels zumindest drei anderen Trockenelektroden der zumindest vier Trockenelektroden der anziehbaren Vorrichtung,
Verarbeiten der erfassten zumindest drei elektrophysiologischen Signale, um das mehrkanalige Elektrokardiogramm des Lebewesens zu erhalten.
